# EUROPEAN PATENT APPLICATION

(11) **EP 4 579 697 A1**
(43) Date of publication of application: **02.07.2025**
(21) Application number: 24223410.2
(22) Date of filing: 27.12.2024
(51) Int. Cl.: H01F 30/12, H01F 38/38, H02M 7/10, H01F 27/30, H01F 38/08, H02M 1/14, H01F 27/40

(54) **HIGH-VOLTAGE GENERATOR AND X-RAY SYSTEM**

(30) Priority: 27.12.2023 CN 202311826474
(71) Applicant: Shanghai United Imaging Healthcare Co., Ltd., Shanghai 201807 (CN)
(72) Inventor: LUO, Biao, Shanghai, 201807 (CN); ZHU, Guoping, Shanghai, 201807 (CN); WU, Jinglin, Shanghai, 201807 (CN)
(74) Representative: Murgitroyd & Company

(57) **Abstract**

The present disclosure provides a high-voltage generator, which includes a high-voltage transformer. The high-voltage transformer includes M magnetic core assembly, M primary windings, and M*N secondary windings. The magnetic core assembly includes M magnetic pillar sets. The M primary windings are respectively wound on the M magnetic pillar sets. N layers of the secondary windings are concentrically wound on each of the primary windings, and M, N are positive integers. The M primary windings are configured such that first ends of the M primary windings receive the input of the M-phase AC power and second ends of the M primary windings are interconnected.

## Description

### TECHNICAL FIELD

The present disclosure relates to the technical field of X-ray systems, particularly to a high-voltage generator and an X-ray system.

### BACKGROUND

High-voltage generators are used to output high-voltage DC power. Ripple is one of the issues that need to be addressed in the high-voltage generator. Particularly during high-voltage output, in order to meet the requirements of the load, such as in a magnetic resonance imaging (MRI) device or an imaging system, a small output ripple is desired.

### SUMMARY

One aspect of the present disclosure provides a high-voltage generator including a high-voltage transformer configured to receive an input of M-phase AC power. The high-voltage transformer includes a magnetic core assembly, M primary windings and M*N secondary windings. The magnetic core assembly includes M magnetic pillar sets. The M primary windings are respectively wound on the M magnetic pillar sets. N layers of the secondary windings are concentrically wound on each of the primary windings, where M ≧ 3, N ≧ 2, and M, N are positive integers. The M primary windings are configured such that first ends of the M primary windings receive the input of the M-phase AC power and second ends of the M primary windings are interconnected.

In some embodiments, the magnetic core assembly includes M magnetic cores. The M magnetic cores each at least include two magnetic pillars, and adjacent magnetic pillars of two adjacent magnetic cores form one of the M magnetic pillar sets, such that the M magnetic cores form the M magnetic pillar sets.

In some embodiments, the M magnetic cores have the same specification parameters.

In some embodiments, the magnetic core is of a symmetrical structure and the two magnetic pillars of the magnetic core are symmetrically arranged with respect to a symmetry axis of the magnetic core.

In some embodiments, the magnetic core is a U-shaped magnetic core or a square magnetic core.

In some embodiments, the M primary windings are wye-connected or delta-connected. N layers of the secondary windings are stacked on each of the magnetic pillar sets. M secondary windings of the i-th layer on the M magnetic pillar sets are wye-connected or delta-connected, where i is 1, 2..., N.

In some embodiments, the high-voltage generator further includes a rectifier circuit. The rectifier circuit includes N rectifier modules. Input terminals of the i-th rectifier module are connected to the M secondary windings of the i-th layer on the M magnetic pillar sets, respectively, and output terminals of the N rectifier modules are connected in series.

In some embodiments, each of the rectifier modules includes a first power output terminal and a second power output terminal. The first power output terminal of a first rectifier module is grounded, the second power output terminal of the j-th rectifier module is connected to the first power output terminal of the (j+1)-th rectifier module, and the second power output terminal of the N-th rectifier module serves as an output terminal of the rectifier circuit, where j is 1, 2..., N.

In some embodiments, the rectifier modules are stacked, with each of the rectifier modules corresponding to one layer. Each of the rectifier modules includes rectifying diodes, and the rectifying diodes of the rectifier module of the j-th layer and the rectifying diodes of the rectifier module of the (j+1)-th layer are orientated in opposite directions.

In some embodiments, the M secondary windings corresponding to a same rectifier module are wye-connected, and the rectifier modules are stacked in a first region enclosed by the magnetic core assembly.

In some embodiments, each of the rectifier modules includes M single-phase rectifier bridge arms connected in parallel between the first power output terminal and the second power output terminal. A second region is formed between the outermost secondary windings of two adjacent magnetic pillar sets, such that the outermost secondary windings of the M magnetic pillar sets form M second regions. The M single-phase rectifier bridge arms of the rectifier module are respectively located in the M second regions. One power output terminal of each of the M single-phase rectifier bridge arms is connected to a connection point located in the first region, and the other power output terminals of the M single-phase rectifier bridge arms are connected at a periphery of the high-voltage transformer to form a voltage equalizing ring.

In some embodiments, the high-voltage generator further includes a multi-phase inverter circuit configured to convert input DC power to M-phase AC power. The multi-phase inverter circuit is connected to the M primary windings.

In some embodiments, the multi-phase inverter circuit includes M switching bridge arms connected in parallel. The M primary windings are connected to the corresponding switching bridge arms.

In some embodiments, the multi-phase inverter circuit further includes M first resonant inductors and M first resonant capacitors. The M primary windings are connected to the corresponding switching bridge arms through the M first resonant capacitors and the M first resonant inductors, respectively.

In some embodiments, the multi-phase inverter circuit further includes M second resonant inductors. The M second resonant inductors are connected in parallel to the M primary windings, respectively.

In some embodiments, the high-voltage generator further includes M*N second resonant capacitors. A first end of each of the second resonant capacitors is connected to an output terminal of the corresponding secondary winding, and second ends of the M second resonant capacitors connected to the M secondary windings in a same layer are interconnected.

In some embodiments, the high-voltage transformer further includes an insulating layer between the primary winding and the secondary windings wound on a same magnetic pillar set.

Another aspect of the present disclosure provides an X-ray system including a high-voltage generator according to any one of the embodiments as described above.

Details of one or more embodiments of the present disclosure are set forth in the following accompanying drawings and descriptions. Other features, objectives, and advantages of the present disclosure become obvious with reference to the specification, the accompanying drawings, and the claims.

### BRIEF DESCRIPTION OF THE DRAWINGS

In order to more clearly explain the technical solutions of the embodiments of the present disclosure, a brief introduction to the drawings required for the description of the embodiments or prior art is provided below. Apparently, the drawings described below are only some of the embodiments of the present disclosure. For those skilled in the art, without making inventive efforts, other drawings can be derived from these drawings.
FIG. 1 is a schematic diagram of a structure of a related high-voltage generator.
FIG. 2 is a schematic diagram of a first structure of a high-voltage transformer according to embodiments of the present disclosure.
FIG. 3 is a schematic diagram of a first structure of a high-voltage transformer and a rectifier circuit according to embodiments of the present disclosure.
FIG. 4 is a schematic diagram of a second structure of a high-voltage transformer and a rectifier circuit according to embodiments of the present disclosure.
FIG. 5 is a schematic diagram of a first circuit of a high-voltage generator according to embodiments of the present disclosure.
FIG. 6 is a schematic diagram of a second circuit of a high-voltage generator according to embodiments of the present disclosure.
FIG. 7 is a schematic diagram of a third circuit of a high-voltage generator according to embodiments of the present disclosure.
FIG. 8 is a schematic diagram of a fourth circuit of a high-voltage generator according to embodiments of the present disclosure.
FIG. 9 is a schematic diagram of a third structure of a high-voltage transformer and a rectifier circuit according to embodiments of the present disclosure.
FIG. 10 is a schematic diagram of a fourth structure of a high-voltage transformer and a rectifier circuit according to embodiments of the present disclosure.
FIG. 11 is a schematic diagram of a fifth structure of a high-voltage transformer and a rectifier circuit according to embodiments of the present disclosure.
FIG. 12 is a schematic diagram of a sixth structure of a high-voltage transformer and a rectifier circuit according to embodiments of the present disclosure.
FIG. 13 is a schematic diagram of a seventh structure of a high-voltage transformer and a rectifier circuit according to embodiments of the present disclosure.

### DETAILED DESCRIPTION

In order to make the technical problems, technical solutions, and beneficial effects to be addressed by the present disclosure clearer, the following further detailed description of the present disclosure is provided in conjunction with the accompanying drawings and embodiments. It should be understood that the specific embodiments described here are merely for explaining the present disclosure and are not intended to limit the present disclosure.

It should be noted that when an element is referred to as being "fixed to" or "arranged on" another element, it can be directly placed on or indirectly placed on the other element. When an element is referred to as being "connected to" another element, it can be either directly connected to the other element or indirectly connected to it.

It should be understood that the terms "length", "width", "up", "down", "front", "back", "left", "right", "vertical", "horizontal", "top", "bottom", "inside", "outside", and other such directional or positional terms refer to the orientation or positional relationships based on the orientations or positions shown in the drawings. These terms are used merely for convenience in describing the present disclosure and simplifying the description, and do not indicate or suggest that the device or element in question must have a specific orientation, be constructed, or operate in a particular orientation. Therefore, they should not be construed as limitations of the present disclosure.

Additionally, the terms "first" and "second" are used only for descriptive purposes and should not be understood as indicating or implying relative importance or the implied number of technical features being referred to. Thus, features labeled as "first", "second", and so on, may explicitly or implicitly include one or more instances of that feature. In the description of the present disclosure, the term "multiple" means two or more, unless otherwise explicitly specified.

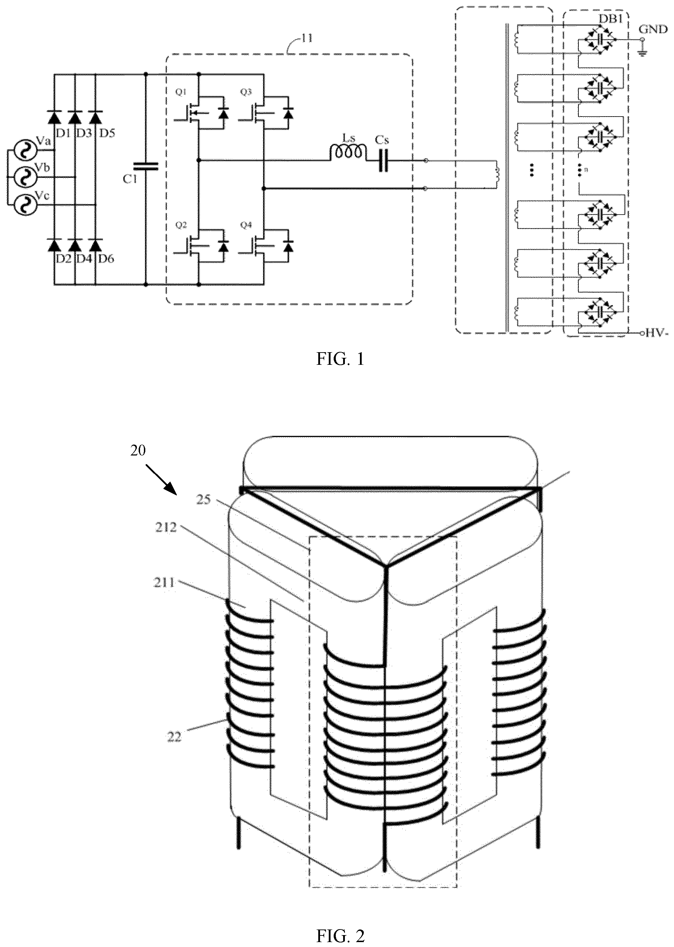

As shown in FIG. 1, a related high-voltage generator includes a single-phase full-bridge inverter circuit 11, a high-voltage transformer, and a rectifier circuit. The single-phase full-bridge inverter circuit 11 inverts the input DC power into high-frequency AC power, which is then transmitted to the high-voltage transformer for voltage step-up through resonant components such as inductors and capacitors. Finally, the rectifier circuit generates a required DC voltage. The primary winding of the high-voltage transformer typically consists of either a single winding or multiple windings in parallel, while multiple secondary windings are present, each corresponding to a single-phase rectifier circuit. The rectifier circuits are connected in series to output a high voltage.

A smaller output ripple can be achieved by increasing the switching frequency or increasing the capacitance of the output filter capacitor. However, increasing the switching frequency will lead to an increase in the losses of the high-voltage generator. Increasing the capacitance will result in a larger size, while also affecting the dynamic response of the output voltage switching speed of the high-voltage generator.

One aspect of the present disclosure provides a high-voltage generator, which includes a high-voltage transformer. The high-voltage transformer includes M magnetic core assembly, M primary windings, and M*N secondary windings. The magnetic core assembly includes M magnetic pillar sets. The M primary windings are respectively wound on the M magnetic pillar sets. N layers of the secondary windings are concentrically wound on each of the primary windings, where M ≧ 3, N ≧ 2, and M, N are positive integers. The M primary windings are configured such that first ends of the M primary windings receive the input of the M-phase AC power and second ends of the M primary windings are interconnected.

As shown in FIGS. 2-4,In some embodiments, the high-voltage generator includes a high-voltage transformer 20 configured to receive an input of AC power, and the high-voltage transformer 20 includes M magnetic cores 21, M primary windings 22, and M*N secondary windings 23. The M magnetic cores 21 constitute the magnetic core assembly.

It can be understood that, in other embodiments, the magnetic core assembly may be a single magnetic core structure, which includes M magnetic pillars, and each individual magnetic pillar constitutes one magnetic pillar set. Alternatively, the single magnetic core structure includes M magnetic pillar pairs, and each magnetic pillar pair constitutes one magnetic pillar set.

The magnetic core assembly including M magnetic cores is taking as an example in the following detailed description. Each of the M magnetic cores 21 at least includes two magnetic pillars 211. Any two magnetic cores 21 are arranged such that magnetic pillars 211 of the magnetic cores 21 are adjacent to each other. The adjacent magnetic pillars 211 of two adjacent magnetic cores 21 form a magnetic pillar set 25, and all the adjacent magnetic pillars 211 of the M magnetic cores 21 form M magnetic pillar sets 25. The M primary windings 22 are wound on the M magnetic pillar sets 25 in a one-to-one correspondence. N layers of the secondary windings 23 are concentrically wound on each of the primary windings 22, where M :> 3, N :> 2, and M, N are positive integers. The M primary windings are configured such that first ends of the M primary windings receive the input of the M-phase AC power and second ends of the M primary windings are interconnected.

In the embodiments, the number of magnetic cores 21 is equal to the number of phases of the AC power supply. The M primary windings 22 of the high-voltage transformer 20 form a multi-phase primary winding structure. Similarly, the secondary windings 23 of the transformer form an N-phase secondary winding structure. The M-phase AC power supply is stepped up by the high-voltage transformer 20 to generate N channels of stepped-up AC power.

The primary windings 22 can be formed by winding materials such as wire-wrapped wire, litz wire, etc., or copper foil. The secondary windings 23 can be formed by winding materials such as wire-wrapped wire or litz wire on a winding frame in layers or slots, or using a PCB winding structure, so as to improve the compactness of the structure.

Ripple refers to the AC component in the DC voltage. In a single-phase rectifier circuit 30, the voltage at a certain point will drop to zero. By using a multi-phase high-voltage transformer 20, the rectified voltage in the multi-phase rectifier circuit 30 connected to the multi-phase high-voltage transformer 20 will not drop to zero. Additionally, the ripple in the multi-phase rectifier circuit 30 has a high frequency and can be filtered out, thereby significantly reducing the ripple.

Therefore, by adjusting the structure of the high-voltage transformer 20 to implement multi-phase voltage step-up, a smaller output ripple can be achieved, along with faster output voltage switching, without the need to change the switching frequency or output capacitance. The switching frequency and output capacitance can be appropriately reduced, which helps to lower switching losses and reduce the size of the high-voltage generator.

At the same time, in order to achieve consistency in the leakage inductance parameters of the high-voltage transformer 20 and reduce the size of the high-voltage transformer 20 and the rectifier circuit 30, in some embodiments, each magnetic core 21 at least includes two magnetic pillars 211. Any two magnetic cores 21 from the M magnetic cores 21 are arranged such that the magnetic pillars 211 of the two magnetic cores 21 are adjacent to each other, and a ring structure is thus formed. The magnetic pillars 211 of two adjacent magnetic cores 21 are adjacent to each other. The two adjacent magnetic pillars 211 form a magnetic pillar set 25, and M magnetic cores 21 form M magnetic pillar sets 25. For example, the high-voltage transformer 20 includes three magnetic cores 21, and each magnetic core 21 includes a first magnetic pillar and a second magnetic pillar. A first magnetic pillar of a first magnetic core 21 is adjacent to a second magnetic pillar of a second magnetic core 21, forming a magnetic pillar set 25. A second magnetic pillar of the second magnetic core 21 is adjacent to the first magnetic pillar of a third magnetic core 21, forming another magnetic pillar set 25. The second magnetic pillar of the third magnetic core 21 is adjacent to the second magnetic pillar of the first magnetic core 21, forming a third magnetic pillar set 25.

The M primary windings 22 are wound on the M magnetic pillars 211 in a one-to-one correspondence. The number of turns and wire diameter of the primary windings 22 are the same.

N layers of the secondary windings 23 are concentrically wound on each primary winding 22. In order to reduce electromagnetic interference between the primary winding 22 and the secondary winding 23, in some embodiments, an insulating layer 24 is provided between the primary winding 22 and the secondary windings 23 wound on the same magnetic pillar set 25, ensuring insulation between the primary winding 22 and the secondary windings 23. Meanwhile, the layers of secondary windings 23 are also insulated from each other using insulating layers 24. Therefore, one layer of primary winding 22 and N layers of secondary windings 23 are wound on each magnetic pillar set 25. Similarly, the number of turns and wire diameter of the secondary windings 23 in the same layer are the same, and the number of turns and wire diameter of adjacent layers of secondary windings 23 can be the same or different.

By arranging the M magnetic cores 21 adjacently in sequence to form a symmetrical ring structure, the consistency in leakage inductance of the high-voltage transformer 20 can be ensured, and the size of the high-voltage transformer 20 and the rectifier circuit 30 is reduced.

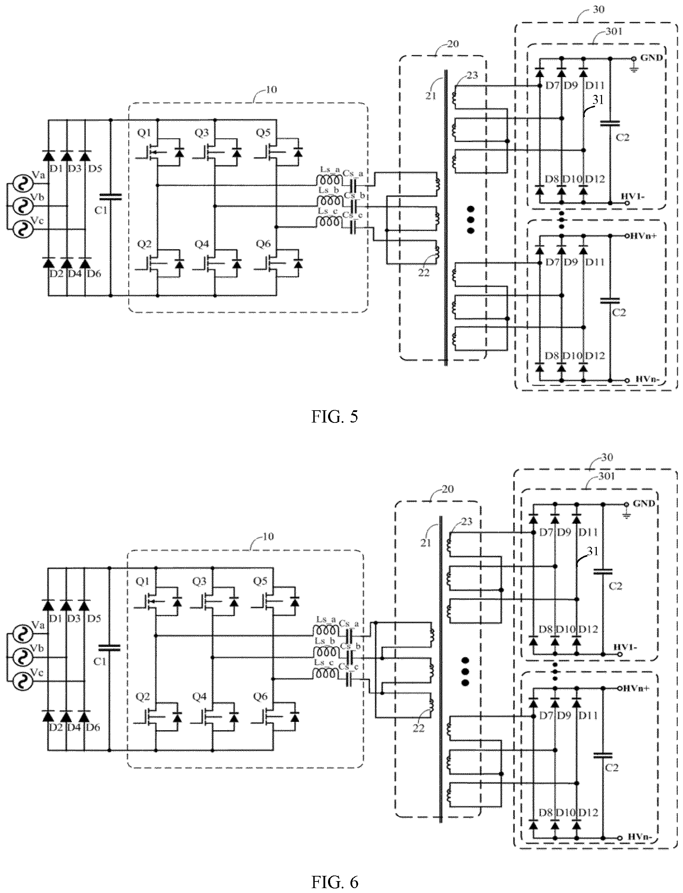

The connection between the primary winding 22 and the secondary winding 23 can be set according to requirements. As shown in FIGS. 5-6, in some embodiments, the M primary windings 22 are wye-connected or delta-connected. The N layers of secondary windings 23 are stacked on each magnetic pillar set 25. The M secondary windings 23 of the i-th layer on the M magnetic pillar sets 25 are wye-connected or delta-connected, where i = 1, 2, ..., N.

For example, the high-voltage transformer 20 includes three magnetic pillar sets 25. The secondary windings 23 of the first layers on the first, second, and third magnetic pillar sets 25 are wye-connected or delta-connected. Similarly, the secondary windings 23 of the second layers on the first, second, and third magnetic pillar sets 25 are wye-connected or delta-connected. In the same way, the secondary windings 23 of the N-th layer on the first, second, and third magnetic pillar sets 25 are wye-connected or delta-connected.

Depending on the connection, the line voltage and phase voltage of the primary winding 22 and secondary windings 23 of each layer vary accordingly.

To further achieve consistency in the leakage inductance parameters of the high-voltage transformer 20, in some embodiments, the specifications of the M magnetic cores 21 are the same, including the size, material, shape, and other specifications, and thus the parasitic parameters generated by the AC power on the M magnetic cores 21 are consistent, thereby achieving leakage inductance consistency.

To achieve symmetry of the ring structure formed by the M magnetic cores 21 to further ensure the consistency in the leakage inductance parameters of the high-voltage transformer 20, in some embodiments, the magnetic cores 21 are of a symmetrical structure, where the two magnetic pillars 211 of the magnetic core 21 are symmetrically arranged with respect to an axis of symmetry of the magnetic core 21. The magnetic core 21 also includes a connecting section 212 that connects the two magnetic pillars 211, and the axis of symmetry is a perpendicular line passing through the midpoint of the connecting section 212. As a result, the M magnetic cores 21, which are arranged adjacent to each other, form a symmetrical ring structure, ensuring that the leakage inductance parameters generated by the AC power on the magnetic pillar sets 25 are consistent. Additionally, due to the symmetrical structure of the magnetic cores 21, the overall size of the high-voltage transformer 20 is further reduced.

In some embodiments, the magnetic core 21 may have different symmetrical structures, such as U-shaped magnetic cores or square magnetic cores. In the embodiments where the magnetic core assembly is of a single magnetic core structure, the shape of the magnetic core assembly may be similar to the entire shape of the M U-shaped magnetic cores or the M magnetic square magnetic cores.

The U-shaped or square magnetic core includes two magnetic pillars 211, and the two magnetic pillars 211 are connected by a connecting section 212. The U-shaped magnetic core has one connecting section 212, while the square magnetic core has two connecting sections 212.

As shown in FIGS. 2-4, taking the U-shaped magnetic core as an example, one magnetic pillar 211 of a U-shaped magnetic core is arranged adjacent to the other magnetic pillar 211 of another U-shaped magnetic core to form a magnetic pillar set 25. The 2M magnetic pillars 211 of the M U-shaped magnetic cores form M magnetic pillar sets 25, such that the M U-shaped magnetic cores are symmetrically arranged, and M primary windings 22 are wound on the M magnetic pillar sets 25 respectively. At the same time, first ends of the M primary windings 22 are connected to an M-phase AC power supply, and the other ends of the M primary windings 22 are wye-connected or delta-connected.

The secondary windings 23 and the primary winding 22 on the same magnetic pillar set 25 are concentrically wound. N layers of secondary windings 23 are stacked on the corresponding primary winding 22. Each layer consists of M secondary windings 23, with first ends of the M secondary windings 23 being led out and connected to a back-end circuit. Second ends of the M secondary windings 23 are wye-connected or delta-connected. An insulating layer 24 is disposed between the primary winding 22 and the secondary windings 23.

The values of M and N can be set according to ripple requirements and output voltage. M can be 3, 4, or even larger. For example, when M is equal to 3, the three magnetic cores 21 are symmetrically arranged in a triangular configuration, and the voltage phases of the three primary windings 22 of the high-voltage transformer 20 are 120 degrees apart from one another. When more windings are present, such as four, the eight magnetic pillars 211 of four magnetic cores 21 are still adjacent to each other in pairs, forming four magnetic pillar sets 25, and the four magnetic cores 21 are symmetrically arranged in a square configuration.

In some embodiments, as shown in FIGS. 2-4, M is equal to 3, i.e., three primary windings 22 and 3N secondary windings are present, meaning that the number of secondary windings 23 in each layer is three. The first ends of the three primary windings 22 are connected to a three-phase AC power supply, and the second ends of the three primary windings 22 are wye-connected or delta-connected. Similarly, the first ends of the three secondary windings 23 in each layer form power output terminals of the high-voltage transformer 20, and the second ends of the three secondary windings 23 in each layer are wye-connected or delta-connected. The specific connection can be set according to the required output voltage and the structural requirements of the transformer.

To achieve the output of high-voltage DC power, as shown in FIGS. 5-6, in some embodiments, the high-voltage generator also includes a rectifier circuit 30, which includes N rectifier modules 301. Input terminals of the i-th rectifier module 301 are connected to the M secondary windings 23 of the i-th layer on the M magnetic pillar sets 25. Output terminals of the N rectifier modules 301 are connected in series, where i is 1, 2..., N.

In these embodiments, the rectifier module 301 is a multi-phase rectifier module. Each rectifier module 301 is connected to M secondary windings 23 and receives the stepped-up M-phase high-frequency AC power. The rectifier modules 301 then rectify the stepped-up M-phase high-frequency AC power to generate corresponding DC voltages. The multiple DC voltages are then combined in series to generate a higher DC voltage, which is output to a backend load.

Additionally, an output capacitor C2 is connected to the backend of each rectifier module 301. By adjusting the structure of the high-voltage transformer 20 and the rectifier circuit 30, multi-phase stepping-up and rectification can be realized, resulting in smaller output ripple and faster output voltage switching, without the need to change the switching frequency or the capacitance of the capacitor C2. The switching frequency and the capacitance of the output capacitor C2 may also be appropriately reduced to lower the switching losses and decrease the size of the switching power supply circuit.

In some embodiments, the i-th rectifier module 301 is connected to the M secondary windings 23 of the i-th layer, for example, the first rectifier module 301 is connected to the M secondary windings 23 of the first layer, the second rectifier module 301 is connected to the M secondary windings 23 of the second layer, and in the same way, the N-th rectifier module 301 is connected to the M secondary windings 23 of the N-th layer.

In some embodiments, the M secondary windings 23 of the first layer are the innermost secondary windings 23, located closest to the primary windings 22, while the M secondary windings 23 of the N-th layer are the outermost secondary windings 23.

Incorrect polarity connections may cause the output voltage of the rectifier module 301 to decrease. For example, when the same output terminals of the rectifier modules 301 are connected in parallel, the output voltage of N rectifier modules 301 will be equal to the output voltage of a single rectifier module 301, resulting in the inability to output DC high voltage. To achieve the summation and amplification of the output voltages, in some embodiments, each rectifier module 301 includes a first power output terminal and a second power output terminal, with the first power output terminal and the second power output terminal being the positive power output terminal HV+ and the negative power output terminal HV-, respectively. This includes two connection scenarios, i.e., a first connection scenario where the first power output terminal is the positive power output terminal HV+ and the second power output terminal is the negative power output terminal HV-, and a second connection scenario where the second power output terminal is the positive power output terminal HV+ and the first power output terminal is the negative power output terminal HV-.

For example, the first power output terminal of the first rectifier module 301 is grounded, the second power output terminal of the j-th rectifier module 301 is connected to the first power output terminal of the (j+1)-th rectifier module 301, and the second power output terminal of the N-th rectifier module 301 serves as the output terminal of the rectifier circuit 30, where j is 1, 2..., N.

For example, the first power output terminal of the first rectifier module 301 is connected to the second power output terminal of a second rectifier module 301, the first power output terminal of the second rectifier module 301 is connected to the second power output terminal of a third rectifier module 301. In this way, the rectifier modules 301 are connected in series, with the output voltages being added together. Meanwhile, the first power output terminal of one rectifier module 301 and the second power output terminal of another rectifier module 301, which are located at two ends of the series, form the power output terminals of the rectifier circuit 30, which output the DC high voltage.

When the first power output terminal is the positive power output terminal HV+ and the second power output terminal is the negative power output terminal HV-, the rectifier circuit outputs a negative DC high voltage. When the first power output terminal is the negative power output terminal HV- and the second power output terminal is the positive power output terminal HV+, the rectifier circuit outputs a positive DC high voltage. The output can be selectively chosen as either negative or positive DC high voltage according to the requirements.

To facilitate the connection between the rectifier modules 301 and simplify the wiring structure, in some embodiments, the rectifier modules 301 are stacked, with each rectifier module 301 corresponding to a layer. Each rectifier module 301 includes rectifying diodes. The rectifying diodes in the rectifier module 301 of the j-th layer and the rectifying diodes in the rectifier module 301 of the (j+1)-th layer are oriented in opposite directions.

Corresponding to the structure of the high-voltage transformer 20, the rectifier modules 301 have a multi-phase structure. In some embodiments, as shown in FIGS. 5-6, each rectifier module 301 includes M single-phase rectifier bridge arms 31 connected in parallel between the first and second power output terminals. Each single-phase rectifier bridge arm 31 is connected in parallel with a respective output capacitor C2, or the three single-phase rectifier bridge arms 31 are connected in parallel with the same output capacitor C2.

The single-phase rectifier bridge arms 31 each include two rectifying diodes, such as rectifying diodes D7 and D8, or rectifying diodes D9 and D10. The 2M rectifying diodes constitute the M-phase rectifier module 301. The connection point of the two rectifying diodes in the single-phase rectifier bridge arm 31 forms the input terminal of the rectifier module 301, while the two ends of the single-phase rectifier bridge arm 31 form the output terminals of the rectifier module 301.

The direction of the rectifying diodes in the rectifier module 301 determines the positive power output terminal HV+ and negative power output terminal HV-. By adjusting the direction of the rectifying diodes, the polarity of the output voltage can be reversed, providing either positive or negative voltage.

Additionally, to facilitate the series connection of an upper and lower layers of rectifier modules 301 for stepping-up of voltage, the output terminals of each rectifier module 301 can be fixed in position. By adjusting the polarity directions of the rectifying diodes in the upper and lower layers, the series connection of rectifier modules 301 becomes easier.

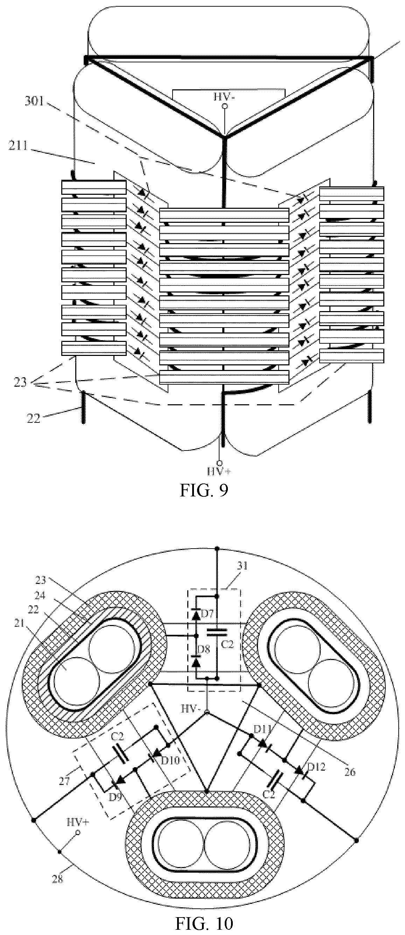

As shown in FIG. 9, after N layers of rectifier modules 301 are connected in series, the first power output terminal of the rectifier module 301 at one end and the second power output terminal of the rectifier module 301 at the other end form the power output terminal of the rectifier circuit 30 and output a DC high voltage. The direction of the rectifying diodes in the rectifier module 301 of each layer determines whether the output voltage of the rectifier module 301 of the N-th layer is positive or negative high voltage.

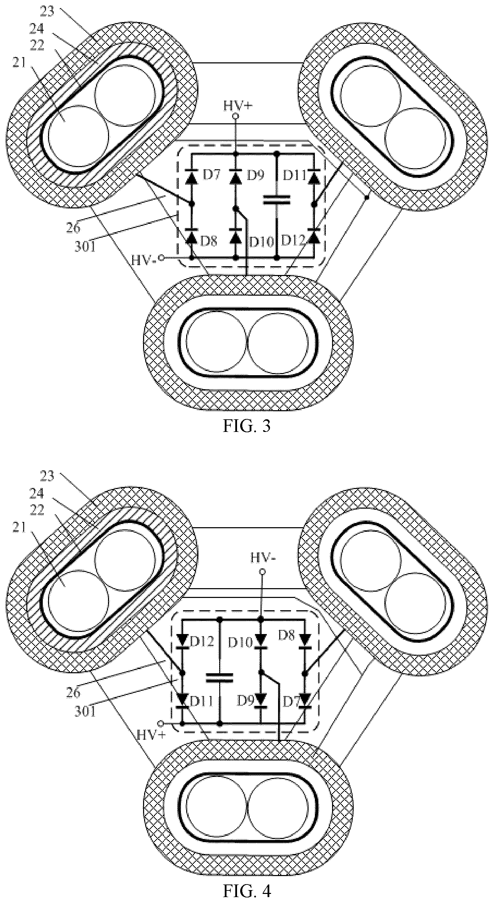

As shown in FIG. 9, rectifier modules 301 are stacked. As shown in FIG. 3, the rectifier module 301 of the j-th layer includes multiple rectifying diodes, with the diodes oriented in a first direction. The two power output terminals of the rectifier module 301 correspond to specific positions on the high-voltage transformer. As shown in FIG. 4, the adjacent rectifier module 301 of the (j+1)-th layer also includes multiple rectifying diodes, but the diodes are oriented in a second direction, opposite to the first direction. Thus, along the stacking direction, the power output terminals of the rectifier modules 301 of the (j+1)-th layer and the j-th layer have opposite polarities, while being positioned adjacently. This configuration allows for direct connection of the first and second power output terminals of two adjacent rectifier modules 301 along the stacking direction using short connecting wires, avoiding the need for cross-wirings. This further simplifies the wiring structure between adjacent layers of rectifier modules 301, prevents issues such as crosstalk caused by cross-wirings, and improves the reliability of voltage conversion.

Furthermore, in order to simplify the overall structure and the size of the high-voltage generator, in some embodiments, as shown in FIG. 3-4, when the M primary windings 22 and the M secondary windings 23 corresponding to the same rectifier module 301 are both wye-connected, the rectifier modules 301 are stacked within the first region 26 enclosed by the magnetic core assembly, for example, by the M magnetic cores 21 in the present embodiment, i.e., each rectifier module 301 is positioned in the region formed by the ring structure of the magnetic cores 21, eliminating the need to arrange the rectifier modules 301 outside the magnetic cores 21 and thereby simplifying the overall structure and reducing the size of the high-voltage generator.

In addition, along the stacking direction, the power output terminals of the rectifier modules 301 of the (j+1)-th layer and the j-th layer have opposite polarities. This configuration allows for direct connection of the first and second power output terminals of two adjacent rectifier modules 301 along the stacking direction using short connecting wires, avoiding the need for cross-wirings. This further simplifies the wiring structure between adjacent layers of rectifier modules 301, prevents issues such as crosstalk caused by cross-wirings, and improves the reliability of voltage conversion.

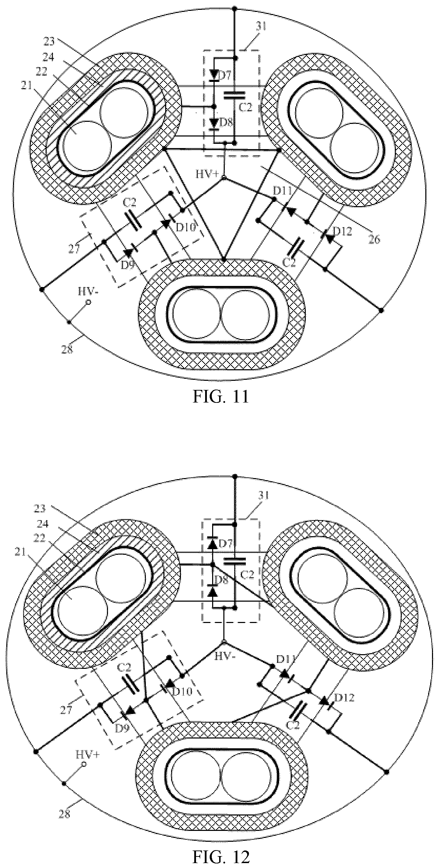

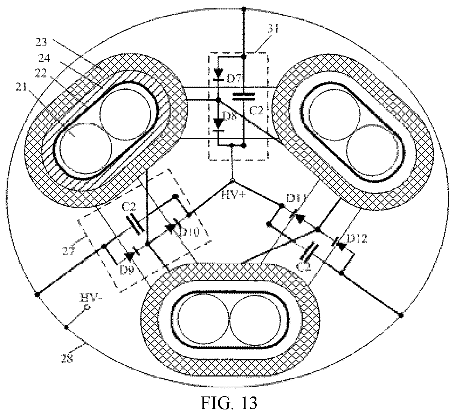

To enhance the reliability of high-voltage insulation, as shown in FIGS. 10-13, in some embodiments, a second region 27 is formed between the outermost secondary windings 23 on adjacent magnetic pillar sets 25. M second regions are thus formed between the outermost secondary windings 23 on the M magnetic pillar sets, and the M single-phase rectifier bridge arms 31 of the rectifier modules 301 are located within the M second regions 27, respectively. One power output terminal of each of the M single-phase rectifier bridge arms 31 is connected to a connection point located in the first region 26, and the other power output terminals of the M single-phase rectifier bridge arms 31 are connected at a periphery of the high-voltage transformer to form a voltage equalizing ring 28. The M secondary windings 23 of the same layer can be wye-connected as shown in FIGS. 10-11, or delta-connected as shown in FIGS. 12-13.

As shown in FIGS. 10-13, for example, M is equal to 3, and the rectifier module 301 includes a first single-phase rectifier bridge arm 31 consisting of diodes D7 and D8, a second single-phase rectifier bridge arm 31 consisting of diodes D9 and D10, and a third single-phase rectifier bridge arm 31 consisting of diodes D11 and D12. The first single-phase rectifier bridge arm 31 is located in the second region 27 between the outermost secondary windings 23 on the first and second magnetic pillar sets 25, the second single-phase rectifier bridge arm 31 is located in the second region 27 between the outermost secondary windings 23 on the second and third magnetic pillar sets 25, and the third single-phase rectifier bridge arm 31 is located in the second region 27 between the outermost secondary windings 23 on the third and first magnetic pillar sets 25.

At the same time, the three power output terminals of the first, second, and third single-phase rectifier bridge arms 31, which have the same polarity, are interconnected, with the connection point located within the first region 26 enclosed by the three magnetic cores 21. The other power output terminals of the first, second, and third single-phase rectifier bridge arms 31, which have the same polarity, are led out to the periphery of the high-voltage transformer 20 and connected to form a voltage equalizing ring 28, which helps improve the reliability of high-voltage insulation.

The power output terminal at the connection point can either be the positive power output terminal HV+ or the negative power output terminal HV-, and the power output terminal forming the voltage equalizing ring 28 can either be the negative power output terminal HV- or the positive power output terminal HV+.

FIGS. 10-11 show the arrangement of the rectifier modules 301 of the j-th and (j+1)-th layers, respectively. Similarly, FIGS. 12-13 also show the arrangement of the rectifier modules 301 of the j-th and (j+1)-th layers, respectively.

Each single-phase rectifier bridge arm 31 in the rectifier module 301 of the j-th layer includes two rectifying diodes, with the diodes oriented in a first direction. The two power output terminals of the single-phase rectifier bridge arm 31 are located in the first and second regions of the high-voltage transformer, respectively. Each single-phase rectifier bridge arm 31 of the rectifier module 301 of the adjacent (j+1)-th layer also includes two rectifying diodes but oriented in a second direction opposite to the first direction. It follows that along the stacking direction, the power output terminals of the single-phase rectifier bridge arm 31in the (j+1)-th layer and the power output terminals of the corresponding single-phase rectifier bridge arm 31 in the j-th layer have opposite polarities, while being arranged adjacently. This allows the first and second power output terminals of the single-phase rectifier bridge arms 31 of two adjacent rectifier modules 301 to be connected directly along the stacking direction using short wires, without requiring cross-wirings, which simplifies the wiring structure between adjacent rectifier modules 301, avoids problems such as crosstalk caused by cross-wirings, and enhances the reliability of voltage conversion.

Furthermore, to achieve the input of AC power, in some embodiments, as shown in FIGS. 5 and 6, the high-voltage generator also includes a multi-phase inverter circuit 10 configured to convert input DC power into M-phase AC power, and the multi-phase inverter circuit 10 is connected to the M primary windings 22, where M is a positive integer greater than or equal to 3.

In this embodiment, the multi-phase inverter circuit 10 performs a multi-phase inversion conversion of the input DC power, generating high-frequency AC power. The high-frequency AC power is then stepped up by the multi-phase high-frequency transformer and then output to the N rectifier modules 301 of the multi-phase rectifier circuit. The rectifier modules 301 rectify the stepped-up AC power and generate corresponding DC voltages. The multiple DC voltages are combined in series to generate a higher DC voltage, which is output to a backend load.

The first ends of the M primary windings 22 are respectively connected to output terminals of the multi-phase inverter circuit 10, while the second ends of the M primary windings 22 are wye-connected or delta-connected.

As shown in FIGS. 5-6, the high-voltage generator further includes a front-end rectification and filtering circuit, which includes diodes D1 to D6. The front-end rectification and filtering circuit performs three-phase rectification and filtering on the input AC power, and the resulting DC power is output to the multi-phase inverter circuit 10. The multi-phase inverter circuit 10 performs a multi-phase inversion conversion of the input DC power, generating high-frequency AC power. The high-frequency AC power is then stepped up by the multi-phase high-frequency transformer and then output to the N rectifier modules 301 of the multi-phase rectifier circuit. The rectifier modules 301 rectify the stepped-up AC power and generate corresponding DC voltages. The multiple DC voltages are combined in series to generate a higher DC voltage, which is output to a backend load.

The multi-phase inverter circuit 10 includes switching transistors, resonant circuits, etc. As shown in FIGS. 5-6, the multi-phase inverter circuit 10 includes M parallel-connected switching bridge arms. The M primary windings 22 are connected to the corresponding switching bridge arms, respectively.

In this embodiment, each switching bridge arm includes two switching transistors. For example, M is equal to 3, and the M parallel switching bridge arms include a first switching bridge arm consisting of switching transistors Q1 and Q2, a second switching bridge arm consisting of switching transistors Q3 and Q4, and a third switching bridge arm consisting of switching transistors Q5 and Q6. The two switching transistors of each switching bridge arm are connected in series between two input terminals of the multi-phase inverter circuit 10. The M primary windings 22 are connected to the corresponding switching bridge arms, respectively. More specifically, each primary winding 22 is connected to a point between the two switching transistors on the corresponding switching bridge arm. For example, the first primary winding 22 is connected to the first switching bridge arm, the second primary winding 22 is connected to the second switching bridge arm, and the third primary winding 22 is connected to the third switching bridge arm. The multi-phase inverter circuit 10 converts DC power into high-frequency AC power, which is then transmitted to the high-voltage transformer 20 for step-up, and finally converted into DC high voltages by the rectifier modules 301.

Furthermore, as shown in FIGS. 5-8, in order to improve conversion efficiency, in some embodiments, the multi-phase inverter circuit 10 also includes M first resonant inductors and M first resonant capacitors. The M primary windings 22 are connected to the corresponding switching bridge arm through the M first resonant capacitors and M first resonant inductors, respectively.

In this embodiment, the M first resonant inductors include resonant inductors Ls_a, Ls_b, Ls_c, etc., and the M first resonant capacitors include resonant capacitors Cs_a, Cs_b, Cs_c, etc. Each switching bridge arm is connected to the primary winding 22 of the high-voltage transformer 20 through one first resonant inductor and one first resonant capacitor. For example, M is equal to 3, the first primary winding is connected sequentially through the first resonant capacitor Cs_a and the first resonant inductor Ls_a to the first switching bridge arm, the second primary winding is connected sequentially through the first resonant capacitor Cs_b and the first resonant inductor Ls_b to the second switching bridge arm, and the third primary winding is connected sequentially through the first resonant capacitor Cs_c and the first resonant inductor Ls_c to the third switching bridge arm.

The multi-phase inverter circuit 10 converts DC power into high-frequency AC power, which is then transmitted to a high-voltage transformer 20 for step-up through a resonant circuit formed by the first resonant inductor and the first resonant capacitor, and finally converted into the DC high voltage by the rectifier circuit 30. Therefore, the conversion efficiency is improved.

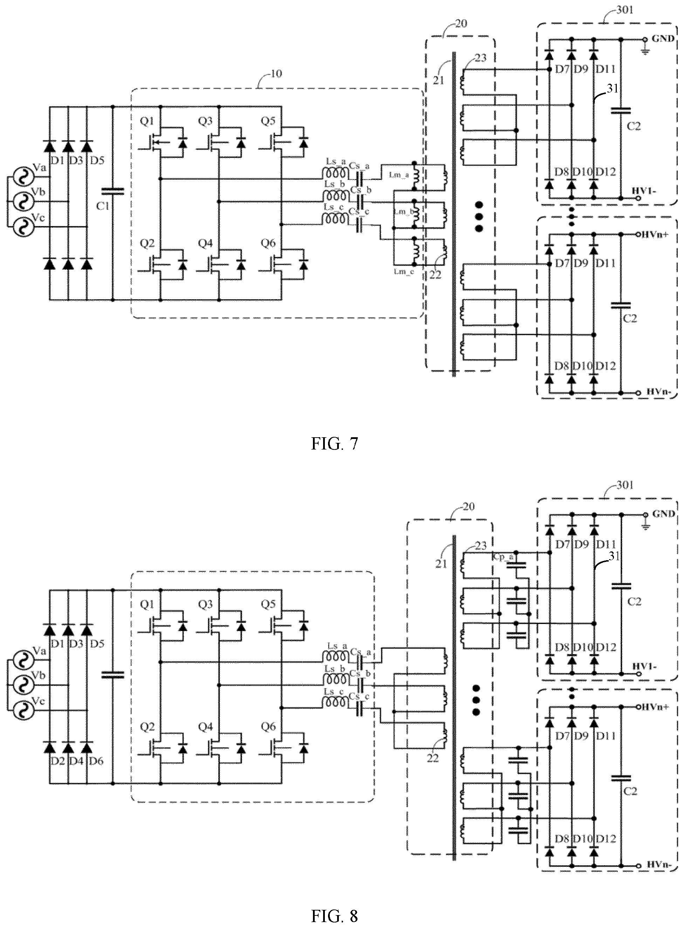

To further improve the conversion efficiency, as shown in FIG. 7, in some embodiments, the multi-phase inverter circuit 10 also includes M second resonant inductors, which are connected in parallel to the M primary windings 22, respectively.

The second resonant inductors include resonant inductors Lm_a, Lm_b, Lm_c, etc. For example, M is equal to 3, the second resonant inductor Lm_a is connected in parallel with the first primary winding 22, the second resonant inductor Lm_b is connected in parallel with the second primary winding 22, and the second resonant inductor Lm_c is connected in parallel with the third primary winding 22. The first resonant inductor, second resonant inductor, and first resonant capacitor in the same path form an LLC resonant circuit, which adjusts the input and output voltages while achieving efficient power conversion.

The second resonant inductors can be additional elements. In some embodiments, the second resonant inductor may be the excitation inductor of the primary winding 22. By utilizing the excitation inductor of the primary winding 22 to form the LLC resonant circuit, the structure of the resonant circuit is simplified, and high-efficiency power conversion is achieved.

As shown in FIG. 8, to increase the output voltage and achieve resonant amplification, in some embodiments, the high-voltage generator also includes M*N second resonant capacitors Cp_a. A first terminal of each second resonant capacitor Cp_a is connected to the output terminal of one secondary winding 23. The second terminals of the M second resonant capacitors Cp_a connected to the secondary windings 23 in the same layer are interconnected.

The second resonant capacitors Cp_a, the first resonant capacitor, and the first resonant inductor form an LCC resonant circuit. Around the resonant point, the second resonant capacitor Cp_a, the first resonant capacitor, and the first resonant inductor form an oscillation loop, where the current will flow back and forth between the second resonant capacitor Cp_a, the first resonant capacitor, and the first resonant inductor, thereby achieving the resonant amplification. The multi-phase inverter circuit 10, by adopting the LCC circuit, realizes soft switching functionality and can adjust the gain by controlling the switching frequency of the multi-phase inverter circuit 10.

The above embodiments illustrate the high-voltage transformer 20 of the present disclosure, using the magnetic core assembly that includes M magnetic cores 21 as an example. It should be noted that the configurations of the primary windings 22, the secondary windings 23, and their connections to the multi-phase inverter circuit 10 and the rectifier current 30, as described above, are also applicable to the magnetic core assembly with a single magnetic core structure, and details will not be repeated here.

Based on various embodiments of the present disclosure, the high-voltage generator includes a high-voltage transformer 20. The high-voltage transformer 20 includes a magnetic core assembly, M primary windings 22, and M*N secondary windings 23. The magnetic core assembly includes M magnetic pillar sets 25. The M primary windings 22 are wound on the M magnetic pillar sets 25 in a one-to-one correspondence. N layers of the secondary windings 23 are concentrically wound on each of the primary windings 22, where M :> 3, N :> 2, and M, N are positive integers. The AC power is stepped up through a multi-phase high-voltage transformer 20, which, with the same switching frequency and output capacitor C2, results in a smaller output ripple. At the same time, the output voltage can be switched rapidly by reducing the capacitance of the output capacitor C2, and the consistency of leakage inductance parameters in the high-voltage transformer 20 is achieved, which helps to reduce the size of the high-voltage transformer 20.

It can be understood that the technical effects summarized above are only for illustrating different embodiments of the present disclosure and do not constitute any limitation on the scope of protection of the present disclosure.

The present disclosure also provides an X-ray system, which includes a high-voltage generator. The specific structure of the high-voltage generator can be obtained with reference to the above-described embodiments. Since the X-ray system adopts the technical solutions of any of the above embodiments, it has at least all the beneficial effects brought by the technical solutions of the above embodiments, which will not be reiterated here.

In the embodiments, the high-voltage generator can be applied in X-ray systems such as magnetic resonance imaging devices and imaging systems, used to provide KV-level DC high voltage. By adopting a high-voltage transformer with a symmetrical structure, the high-voltage generator achieves a smaller output ripple under the same switching frequency and capacitance of the output capacitor C2. At the same time, the capacitance C2 of the output capacitor C2 can be reduced, thereby achieving a fast dynamic response for the high-voltage generator's KV-level change, and ensuring consistency in the leakage inductance parameters of the high-voltage transformer, which reduces the size of the high-voltage transformer.

The above-described embodiments are intended to illustrate the technical solutions of the present disclosure, rather than to limit them. Although the present disclosure has been described in detail with reference to the foregoing embodiments, those skilled in the art should understand that they can still modify the technical solutions described in the previous embodiments, or make equivalent substitutions to some of the technical features. Such modifications or substitutions do not depart from the essence of the technical solutions of the embodiments of the present disclosure, and should all be included within the scope of protection of the present disclosure.

## Claims

1. A high-voltage generator, comprising a high-voltage transformer (20) configured to receive an input of M-phase AC power, **characterized in that** the high-voltage transformer (20) comprises a magnetic core assembly, M primary windings (22) and M*N secondary windings (23), the magnetic core assembly comprises M magnetic pillar sets (25), the M primary windings (22) are respectively wound on the M magnetic pillar sets (25), N layers of the secondary windings (23) are concentrically wound on each of the primary windings (22), where M ≧ 3, N ≧ 2, and M, N are positive integers, and the M primary windings (22) are configured such that first ends of the M primary windings (22) receive the input of the M-phase AC power and second ends of the M primary windings (22) are interconnected.

2. The high-voltage generator of claim 1, wherein the magnetic core assembly comprises M magnetic cores (21), the M magnetic cores (21) each at least comprise two magnetic pillars (211), adjacent magnetic pillars (211) of two adjacent magnetic cores (21) form one of the M magnetic pillar sets (25), such that the M magnetic cores (21) form the M magnetic pillar sets (25).

3. The high-voltage generator of claim 2, wherein the magnetic core (21) is of a symmetrical structure and the two magnetic pillars (211) of the magnetic core (21) are symmetrically arranged with respect to a symmetry axis of the magnetic core (21).

4. The high-voltage generator of claim 3, wherein the magnetic core (21) is a U-shaped magnetic core or a square magnetic core.

5. The high-voltage generator of claim 1, wherein the M primary windings (22) are wye-connected or delta-connected, N layers of the secondary windings (23) are stacked on each of the magnetic pillar sets (25), and M secondary windings (23) of the i-th layer on the M magnetic pillar sets (25) are wye-connected or delta-connected, where i is 1, 2..., N.

6. The high-voltage generator of claim 5, further comprising a rectifier circuit (30), wherein the rectifier circuit (30) comprises N rectifier modules (301), input terminals of the i-th rectifier module (301) are connected to the M secondary windings (23) of the i-th layer on the M magnetic pillar sets (25), respectively, and output terminals of the N rectifier modules (301) are connected in series, and wherein each of the rectifier modules (301) comprises a first power output terminal and a second power output terminal, the first power output terminal of a first rectifier module (301) is grounded, the second power output terminal of the j-th rectifier module (301) is connected to the first power output terminal of the (j+1)-th rectifier module (301), and the second power output terminal of the N-th rectifier module (301) serves as an output terminal of the rectifier circuit (30), where j is 1, 2..., N.

7. The high-voltage generator of claim 6, wherein the N rectifier modules (301) are stacked, with each of the rectifier modules (301) corresponding to one layer, each of the rectifier modules (301) comprises rectifying diodes, and the rectifying diodes of the rectifier module (301) of the j-th layer and the rectifying diodes of the rectifier module (301) of the (j+1)-th layer are orientated in opposite directions.

8. The high-voltage generator of claim 7, wherein the M secondary windings (23) corresponding to a same rectifier module (301) are wye-connected, and the rectifier modules (301) are stacked in a first region (26) enclosed by the magnetic core assembly.

9. The high-voltage generator of claim 7, wherein each of the rectifier modules (301) comprises M single-phase rectifier bridge arms (31) connected in parallel between the first power output terminal and the second power output terminal, a second region (27) is formed between the outermost secondary windings (23) of two adjacent magnetic pillar sets (25), such that the outermost secondary windings (23) of the M magnetic pillar sets (25) form M second regions (27), the M single-phase rectifier bridge arms (31) of the rectifier module (301) are respectively located in the M second regions (27), one power output terminal of each of the M single-phase rectifier bridge arms (31) is connected to a connection point located in the first region (26), and the other power output terminals of the M single-phase rectifier bridge arms (31) are connected at a periphery of the high-voltage transformer (20) to form a voltage equalizing ring (28).

10. The high-voltage generator of claim 5, further comprising a multi-phase inverter circuit configured to convert input DC power to M-phase AC power, the multi-phase inverter circuit being connected to the M primary windings (22),
wherein the multi-phase inverter circuit comprises M switching bridge arms connected in parallel, and the M primary windings (22) are connected to the corresponding switching bridge arms.

11. The high-voltage generator of claim 10, wherein the multi-phase inverter circuit further comprises M first resonant inductors and M first resonant capacitors, and the M primary windings (22) are connected to the corresponding switching bridge arms through the M first resonant capacitors and the M first resonant inductors, respectively.

12. The high-voltage generator of claim 11, wherein the multi-phase inverter circuit further comprises M second resonant inductors, and the M second resonant inductors are connected in parallel to the M primary windings (22), respectively.

13. The high-voltage generator of claim 1, wherein the high-voltage generator further comprises M*N second resonant capacitors, a first end of each of the second resonant capacitors is connected to an output terminal of the corresponding secondary winding, and second ends of the M second resonant capacitors connected to the M secondary windings (23) in a same layer are interconnected.

14. The high-voltage generator of claim 1, wherein the high-voltage transformer (20) further comprises an insulating layer (24) between the primary winding and the secondary windings (23) wound on a same magnetic pillar set (25).

15. An X-ray system, comprising a high-voltage generator, **characterized in that** the high-voltage generator comprises a high-voltage transformer (20) according to any one of claims 1-14.
